# DEMANDE DE BREVET EUROPEEN

(11) **EP 2 392 377 A1**
(43) Date de publication de la demande: **07.12.2011**
(21) Numéro de dépôt: 11168439.5
(22) Date de dépôt: 01.06.2011
(51) Int. Cl.: A61M 25/09, A61M 25/01

(54) **Outil de guidage pour catheter**

(30) Priorité: 03.06.2010 FR 1054373
(71) Demandeur: Somedics RD, 92441 Issy-les-Moulineaux (FR)
(72) Inventeur: Boyer, Jean-Michel, 02400 Nesles la Montagne (FR)
(74) Mandataire: Lebkiri, Alexandre

(57) **Abrégé**

L'invention concerne un outil de guidage (1) pour cathéter. L'outil de guidage (1) selon l'invention comporte notamment :
- une poignée (2) de maintien dudit outil de guidage (1), ladite poignée (2) comportant une cavité interne (6) s'étendant entre une extrémité proximale et une extrémité distale de ladite poignée (2) ;
- un fil guide (4) flexible ;
- un capuchon (3) obturant ladite extrémité proximale de ladite poignée (2).

Ladite extrémité proximale dudit fil guide (4) forme un ressort hélicoïdal (5), ledit ressort hélicoïdal (5) étant positionné dans ladite cavité interne (6) de ladite poignée (2) et maintenu en rotation par ledit capuchon (3).
Une application particulièrement intéressante de l'invention concerne le domaine des stimulateurs cardiaques.

## Description

L'invention concerne un outil de guidage, également désigné par le terme stylet, pour cathéter. L'invention concerne plus particulièrement un outil de guidage et de positionnement d'une sonde de stimulateur cardiaque. En particulier, un tel outil de guidage permet de positionner une sonde de stimulateur cardiaque à un endroit précis du coeur.

Lors de l'implantation d'un stimulateur cardiaque et d'une ou deux sondes, après une anesthésie locale, une incision est réalisée :
- dans le sillon delto-pectoral si l'on aborde la veine céphalique, ou
- en direction de la partie médiane de la clavicule, ce qui permet l'abord de la veine céphalique ou de la ponction sous-clavière.

L'extrémité distale de la sonde est introduite dans la veine choisie jusqu'à être positionnée dans la cavité cardiaque désirée (oreillette ou ventricule).

Une sonde électrode de stimulation cardiaque permanente est habituellement constituée :
- d'un conducteur spiralé isolé de l'environnement extérieur par une gaine faite de silicone ou de polyuréthane ; ou
- de deux conducteurs spiralés parfois isolés indépendamment les uns des autres. Ces conducteurs sont isolés de l'environnement extérieur par une gaine faite de silicone ou de polyuréthane.

Que la technologie utilisée pour la construction de la sonde soit coaxiale ou colinéaire, l'intérieur des conducteurs présente l'aspect d'un tunnel par lequel on introduit l'outil de guidage nommé stylet. L'outil de guidage comporte un fil métallique de diamètre constant généralement compris entre 0,35 à 0,40 mm. L'extrémité proximale de l'outil de guidage étant équipée d'une poignée de manipulation également dénommée « retainer ».

Lors du positionnement d'une sonde cardiaque, le fil guide est introduit en coulissement dans le tunnel interne de courbes serrées de la sonde (conducteur spiralé). L'extrémité distale du fil guide se heurte parfois à des bifurcations formées par la sonde. Ceci est en particulier dû à la matière métallique du fil guide qui lui confère une flexibilité limitée.

En conséquence, lorsque l'extrémité distale du fil guide se heurte à un obstacle formé par la sonde et que sa flexibilité ne lui permet pas de passer malgré l'effort de pression exercé par le praticien, il peut arriver que l'extrémité distale du fil guide transperce dans un premier temps l'enroulement conducteur en détériorant ses spires puis, dans un second temps, perfore la gaine isolante pour ensuite venir au contact d'une veine ou d'un tissu biologique.

Par ailleurs, avec un outil de guidage classique, le praticien glisse ce dernier dans la sonde de stimulateur cardiaque jusqu'à ce que l'extrémité distale de l'outil de guidage soit au contact de l'extrémité distale de la sonde. Lorsque les deux extrémités sont en contact l'une de l'autre, une distance déterminée, sensiblement égale à 20 mm, est présente entre l'entrée de la sonde et l'extrémité distale de la poignée. Il est fréquent que le praticien augmente son effort de poussée jusqu'à ce que la poignée de l'outil de guidage vienne au contact de l'entrée de la sonde. La sonde subit alors une élongation critique.

Dans ce contexte, l'invention vise à proposer un outil de guidage pour cathéter permettant de résoudre les problèmes de l'art antérieur précités en assurant notamment le guidage et le positionnement d'un cathéter de façon sécurisée.

A cette fin, l'objet de l'invention concerne un outil de guidage pour cathéter comportant :
- une poignée de maintien dudit outil, ladite poignée comportant une cavité interne s'étendant entre une extrémité proximale et une extrémité distale de ladite poignée ;
- un fil guide flexible ;
- un capuchon obturant ladite extrémité proximale de ladite poignée ;
ledit outil de guidage étant caractérisé en ce que l'extrémité proximale dudit fil guide forme un ressort hélicoïdal, ledit ressort hélicoïdal étant positionné dans ladite cavité interne de ladite poignée et maintenu en rotation par ledit capuchon, ledit capuchon étant solidaire en rotation de ladite poignée.

En d'autres termes, le capuchon permet de solidariser en rotation la poignée et le ressort hélicoïdal. Ainsi, lorsque la poignée est soumise à un mouvement de rotation, le ressort hélicoïdal et donc le fil guide est également soumis à un mouvement de rotation.

Pour la suite de la description, on entend par cathéter ou sonde, un instrument en forme de tige creuse flexible pouvant être introduite dans un conduit naturel ou un vaisseau sanguin.

De façon conventionnelle, pour toute la description, on entend par :
- extrémité proximale d'un élément : l'extrémité la plus proche de l'extrémité du fil guide destinée à être insérée dans un cathéter ;
- extrémité distale du même élément : l'extrémité la plus éloignée de l'extrémité du fil guide destinée à être insérée dans un cathéter.

Lorsque le fil guide est passé au travers d'un cathéter et qu'il se heurte à un coude, le praticien va accroître son effort de poussée de façon à ce que le fil guide passe cet obstacle. Grâce à l'invention, le ressort formé par l'extrémité proximale du fil guide compense l'effort de poussée de façon à ne pas détériorer la gaine conductrice, la gaine isolante et les tissus biologiques. Une telle compensation est possible grâce à la présence du ressort hélicoïdal. On utilise un ressort hélicoïdal car ce type de ressort particulier permet d'obtenir des courses importantes, d'absorber les efforts auxquels il est soumis et de retrouver sa position initiale lorsqu'il n'est plus soumis à un effort.

Comme le ressort est maintenu en rotation par rapport à la poignée au moyen du capuchon, la poignée de maintien peut être utilisée pour faire pivoter le fil guide dans le cathéter de façon à passer plus aisément les bifurcations que l'extrémité distale du fil guide peut rencontrer.

En résumé, lors de l'insertion du fil guide dans un cathéter, le ressort hélicoïdal et le maintien en rotation du fil guide par rapport à la poignée confèrent à l'outil de guidage selon l'invention une grande facilité d'utilisation permettant de passer les bifurcations imposées par le réseau veineux, formées par le cathéter.

Avantageusement, la matière du fil guide, la course du ressort hélicoïdal et le nombre de spires de ce dernier sont choisis en fonction de la résistance à l'élongation du cathéter.

De préférence, le ressort hélicoïdal formé par le fil guide présente une course d'au moins 20 mm.

Outre les caractéristiques principales qui viennent d'être mentionnées dans le paragraphe précédent, l'outil de guidage pour cathéter selon l'invention peut présenter une ou plusieurs caractéristiques supplémentaires ci-dessous, considérées individuellement ou selon toutes les combinaisons techniquement réalisables :
- ledit capuchon comporte :
   - une première partie sensiblement cylindrique apte à coopérer avec ladite extrémité proximale de ladite poignée ;
   - une deuxième partie formant une plaque sensiblement parallélépipédique située dans le prolongement de ladite première partie de sorte que l'extrémité proximale dudit ressort hélicoïdal puisse être positionnée entre la surface périphérique de ladite cavité interne et la surface externe de ladite deuxième partie ;
- ledit fil guide comporte une gorge au niveau de son extrémité distale, cette gorge augmentant la souplesse de l'extrémité distale du fil guide de sorte que cette dernière puisse aisément suivre les bifurcations du cathéter ;
- ledit fil guide formant le fond de ladite gorge comporte un diamètre sensiblement égale à 0,25 mm ;
- ladite gorge comporte une longueur comprise entre 30 mm et 35 mm ;
- ladite extrémité distale dudit fil guide comporte une tête en forme d'ogive, la surface périphérique de la tête en forme d'ogive permet de guider l'extrémité distale du fil guide lors de son insertion dans le cathéter ;
- ladite tête en forme d'ogive possède une longueur de l'ordre de 4 mm.

D'autres caractéristiques et avantages de l'invention ressortiront clairement de la description qui en est donnée ci-après, à titre indicatif et nullement limitatif, en référence aux figures annexées ci-jointes parmi lesquelles :
- la figure 1 représente un outil de guidage pour cathéter conforme à l'invention ;
- les figures 2A et 2B illustrent un capuchon utilisé dans l'outil de guidage pour cathéter tel que représenté en figure 1 ;
- la figure 3 illustre un outil de guidage tel que représenté sur la figure 1 et mis en oeuvre au sein d'une sonde cardiaque ;
- la figure 4 représente une variante d'un outil de guidage conforme à l'invention ;
- la figure 5 représente une autre variante d'un outil de guidage selon l'invention.

Pour des raisons de clarté, seuls les éléments essentiels pour la compréhension de l'invention ont été représentés, et ceci sans respect de l'échelle et de manière schématique. En outre, les éléments identiques qui se retrouvent sur différentes figures portent les mêmes références.

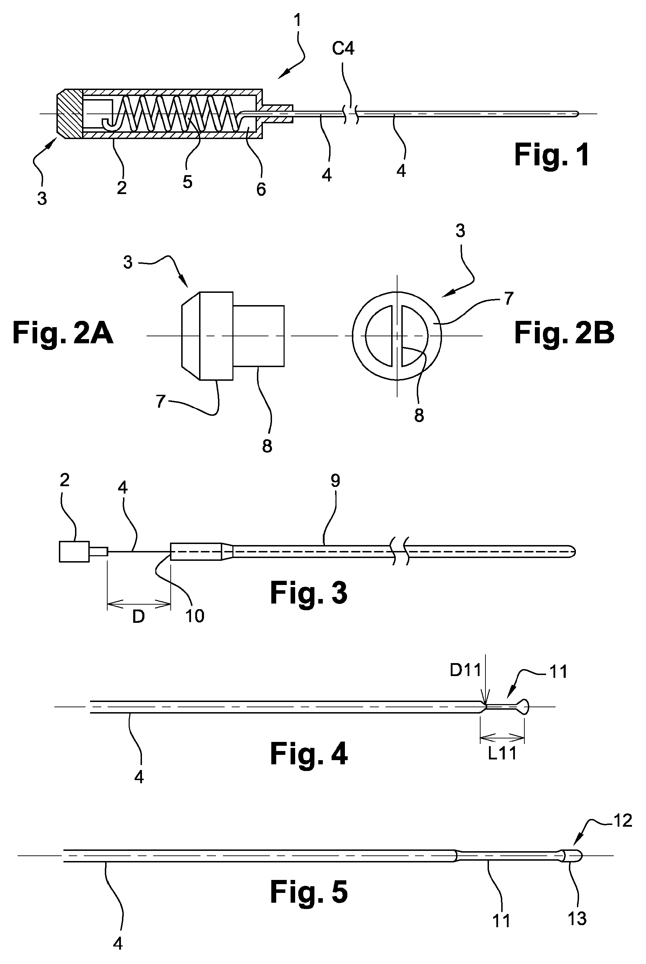

La figure 1 représente un outil de guidage 1 pour cathéter conforme à l'invention. L'outil de guidage 1 comporte notamment :
- une poignée 2 de maintien de l'outil de guidage 1 ;
- un capuchon 3 ;
- un fil guide 4 flexible. Pour la clarté du dessin, afin de pouvoir représenter les parties distale et proximale du fil guide 4, celui-ci est représenté avec une coupure C4.

L'extrémité proximale du fil guide 4 est enroulée de façon à former un ressort hélicoïdal 5.

La poignée 2 comporte une cavité interne 6 s'étendant entre son extrémité proximale et son extrémité distale et est adaptée pour recevoir le ressort hélicoïdal 5.

La cavité interne 6 est débouchante au niveau de l'extrémité proximale de la poignée 2.

De façon à contenir le ressort hélicoïdal 5 dans la cavité interne 6, cette dernière est obturée par le capuchon 3.

Outre le fait de contenir le ressort hélicoïdal 5 dans la cavité interne 6, le capuchon permet également de solidariser en rotation la poignée 2 et le ressort hélicoïdal 5.

Pour ce faire, comme illustré sur la figure 2, le capuchon 3 peut comporter :
- une première partie 7 sensiblement cylindrique apte à coopérer avec l'extrémité proximale de la poignée 2 ;
- une deuxième partie 8 formant une plaque sensiblement parallélépipédique perpendiculaire et au contact de l'extrémité distale de la première partie 7 de sorte que l'extrémité proximale du ressort hélicoïdal 5 puisse être insérée entre la surface périphérique de la cavité interne 6 et la surface externe de la deuxième partie 8.

Cet agencement particulier permet de maintenir en rotation le ressort hélicoïdal 5 par rapport à la poignée 2. Comme on peut le voire sur la figure 1, l'extrémité proximale du ressort hélicoïdal 5 est glissée sous la deuxième partie 8 formant une plaque.

Avantageusement, lors de l'insertion du fil guide 4 au sein d'une sonde cardiaque (non représentée), l'utilisateur peut, grâce au maintien en rotation du fil guide 4 par rapport à la poignée 2, faire pivoter le fil guide 4 en opérant une rotation de la poignée 2. Cette rotation du fil guide 4 permet au praticien de faire passer certaines bifurcations formées par la sonde cardiaque dans lesquelles peut se heurter l'extrémité distale du fil guide 4.

Par ailleurs, à titre d'exemple non limitatif, de façon à confiner le ressort hélicoïdal 5 au sein de la cavité interne 6, la première partie 7 du capuchon 3 peut être vissée dans la poignée 2. Dans une réalisation différente, la première partie 7 du capuchon 3 peut être collée avec la poignée 2.

Comme précédemment indiqué, l'outil de guidage 1 selon l'invention est adapté pour être inséré dans un cathéter et plus particulièrement dans une sonde cardiaque pour le positionnement de cette dernière dans une zone désirée du coeur. Une sonde cardiaque est généralement passée au travers du réseau veineux présentant des bifurcations.

Lorsque l'extrémité distale de la sonde vient au contact d'une bifurcation, le praticien exerce, sur la poignée 2 de l'outil de guidage 1, un effort de poussée plus important de façon à ce que l'extrémité distale de la sonde puisse passer cette bifurcation veineuse. Grâce à l'invention, aucun risque d'endommagement de la sonde ni même de la veine n'est présent car le ressort hélicoïdale 5 vise à compenser un tel excès de poussée.

Le positionnement d'une sonde 9 pour stimulateur cardiaque est représenté plus en détails sur la figure 3.

La sonde 9 est creuse de façon à pouvoir recevoir le fil guide 4. Le fil guide 4 est alors glissé dans la sonde 9 jusqu'à ce que l'extrémité distale du fil guide 4 soit au contact de l'extrémité distale de la sonde 9. Lorsque les deux extrémités sont en contact l'une de l'autre, une distance déterminée D, sensiblement égale à 20 mm, est présente entre l'entrée 10 de la sonde 9 et l'extrémité distale de la poignée 2.

Si le praticien augmente son effort de poussée, le ressort hélicoïdal 5 compensera l'excès de force de façon à ce que la sonde 9 ne subisse pas une élongation. En d'autres termes, c'est le ressort hélicoïdal 5 qui subit une déformation en se comprimant et non la sonde 9.

Une réalisation avantageuse de l'extrémité distale d'un fil guide 4 que comporte un outil de guidage 1 selon l'invention est représentée sur la figure 4.

Plus précisément, dans une telle réalisation, le fil guide 4 est pourvu d'une gorge 11 à son extrémité distale.

Cette gorge 11 confère à l'extrémité distale du fil guide 4 une souplesse importante lui permettant, lors de son insertion, d'épouser les éventuelles bifurcations du cathéter au sein duquel il est inséré.

A titre d'exemple non limitatif, le diamètre du fil guide 4 selon l'invention présente un diamètre de 0,35 mm et le diamètre D11 formant le fond de la gorge 11 est sensiblement égal à 0,25 mm.

En outre, la longueur L11 de la gorge 11 est de préférence comprise entre 30 mm et 35 mm.

La gorge 11 peut être réalisée au moyen d'une opération de tournage ou d'une opération de meulage consistant à retirer de la matière au fil guide 4 pour obtenir un diamètre D11 et une longueur L11 déterminés.

La figure 5 illustre une autre variante d'un fil guide 4 que comporte un outil de guidage 1 conforme à l'invention.

Pour accroître la facilité de passage de l'outil de guidage 1 dans un cathéter, le fil guide 4 est muni à son extrémité distale d'une tête 12 en forme d'ogive.

La surface périphérique 13 de la tête 12 en forme d'ogive permet à cette dernière de se mouvoir aisément au sein d'un cathéter.

Pour ce faire, la tête 12 présente préférentiellement une longueur de l'ordre de 4 mm. Une telle longueur de la tête 12 lui permet d'éviter de passer au travers des spires de la sonde du stimulateur cardiaque.

L'outil de guidage pour cathéter selon l'invention à été plus particulièrement décrit dans le cas d'une utilisation pour le positionnement d'une sonde de stimulateur cardiaque. Toutefois, il est entendu que cet outil de guidage peut être utilisé pour tout type de cathéter.

L'invention est décrite dans ce qui précède à titre d'exemple et il est entendu que l'homme du métier est susceptible de réaliser différentes variantes notamment en ce qui concerne les dimensions du ressort hélicoïdal, de la gorge ou de la tête en forme d'ogive que comporte le fil guide ou encore de la forme du capuchon sans pour autant sortir de cadre de l'invention.

## Revendications

1. Outil de guidage (1) pour cathéter comportant :
- une poignée (2) de maintien dudit outil de guidage (1), ladite poignée (2) comportant une cavité interne (6) s'étendant entre une extrémité proximale et une extrémité distale de ladite poignée (2) ;
- un fil guide (4) flexible ;
- un capuchon (3) obturant ladite extrémité proximale de ladite poignée (2) ;
ledit outil de guidage (1) étant **caractérisé en ce que** l'extrémité proximale dudit fil guide (4) forme un ressort hélicoïdal (5), ledit ressort hélicoïdal 5) étant positionné dans ladite cavité interne (6) de ladite poignée (2) et maintenu en rotation par ledit capuchon (3), ledit capuchon (3) étant solidaire en rotation de ladite poignée (2).

2. Outil de guidage (1) selon la revendication précédente **caractérisé en ce que** ledit ressort hélicoïdal (5) possède une course d'au moins 20 mm.

3. Outil de guidage (1) selon l'une des revendications précédentes **caractérisé en ce que** ledit capuchon (3) comporte :
- une première partie (7) sensiblement cylindrique apte à coopérer avec ladite extrémité proximale de ladite poignée (2) ;
- une deuxième partie (8) formant une plaque sensiblement parallélépipédique située dans le prolongement de ladite première partie (7) de sorte que l'extrémité proximale dudit ressort hélicoïdal (5) puisse être positionnée entre la surface périphérique de ladite cavité interne (6) et la surface externe de ladite deuxième partie (8).

4. Outil de guidage (1) selon l'une des revendications précédentes **caractérisé en ce que** ledit fil guide (4) comporte une gorge (11) au niveau de son extrémité distale.

5. Outil de guidage (1) selon la revendication 4 **caractérisé en ce que** ledit fil guide (4) formant le fond de ladite gorge (11) comporte un diamètre (D11) sensiblement égale à 0,25 mm.

6. Outil de guidage (1) selon l'une des revendications 4 ou 5 **caractérisé en ce que** ladite gorge (11) comporte une longueur (L11) comprise entre 30 mm et 35 mm.

7. Outil de guidage (1) selon l'une des revendications précédentes **caractérisé en ce que** ladite extrémité distale dudit fil guide (4) comporte une tête (12) en forme d'ogive.

8. Outil de guidage (1) selon la revendication 7 **caractérisé en ce que** ladite tête (12) en forme d'ogive possède une longueur de l'ordre de 4 mm.
